# EUROPEAN PATENT SPECIFICATION

(11) **EP 1 028 659 B1**
(45) Date of publication and mention of the grant of the patent: **18.02.2004**
(21) Application number: 97950099.8
(22) Date of filing: 05.11.1997
(51) Int. Cl.: A61B 17/17, A61B 5/107, A61B 5/103

(54) **VIRTUAL REPRESENTATION OF A BONE OR A BONE JOINT**
VIRTUELLE DARSTELLUNG EINES KNOCHENS ODER EINES KNOCHENGELENKES
REPRESENTATION VIRTUELLE D'UN OS OU D'UNE ARTICULATION

(43) Date of publication of application: 23.08.2000
(73) Proprietor: SYNTHES AG CHUR, 7002 Chur (CH)
(72) Inventor: SATI, Marwan, CH-4600 Olten (CH)
(74) Representative: Lusuardi, Werther Giovanni, Dr.
(86) International application number: PCT/EP1997/006107
(87) International publication number: WO 1999/023956

(56) References cited:
- EP-A- 0 603 089
- US-A- 5 682 886

## Description

The invention relates to a device to virtually represent a bone or a bone joint according to the preamble of claim 1.

A method for computer-assisted knee anterior cruciate ligament reconstruction is known from DESSENNE et al, Computer-Assisted Knee Anterior Cruciate Ligament Reconstruction: First Clinical Tests, Journal of Image Guided Surgery 1:59-64 (1995). The chosen procedure is the patellar tendon bone autograft, whereby the method allows positioning the central part of the ligament graft at the least anisometric sites. A perfect isometry implies that there is no change in the distance between the ligament attachment points at the femur and at the tibia. An anisometry is said to exist when there is a change in the distance during knee flexion and extension. With weak anisometry, the graft is subjected to nearly constant tensile forces. Therefore, the risk of rupture because of excessive tensile force in extension or flexion is reduced and the knee stability is improved. The system involved uses a workstation and a three-dimensional optical localizer to create images that represent knee kinematics. This surgical procedure can be performed in a classical open surgery or under use of an arthroscope. The success of the reconstruction depends on both the selection of the intraarticular graft position and the initial graft tension. If the insertion sites, initial tension, geometry and mechanical properties of the normal Anterior Cruciate Ligament can be restored during reconstructive surgery, the long-term complications of an Anterior Cruciate Ligament injury can be greatly reduced. To determine the optimal placement of an anterior cruciate ligament graft, the concept of "isometry" has been advocated by many authors. In the described version of the system, the surgeon drills the tibia tunnel without using the computer system. The system is used to optimize the placement of the femoral tunnel only. The method is divided into four steps:
1) A passive flexion-extension is applied to the knee by the surgeon and for about 20-50 knee positions ranging from maximal extension to maximal flexion. At each position the location of two coordinate systems represented by optical bodies that are fixed to the femur and the tibia are computed and stored.
2) A third optical pointer is used by the surgeon to interactively collect surface points arthroscopically. Once the tibia tunnel has been developed, the center of its intraarticular extremity is digitized with the pointer. Then the surgeon acquires surface points on the femoral notch. In an area that corresponds to all possible candidate points for the femoral attachment site a set of 20-100 points is digitized.
3) Anisometry maps are then computed. The result is an "anisometry map" on the femoral surface that can be presented to the surgeon as a pseudocolor image.
4) This step concludes the interactive placement of the femoral tunnel. The surgeon can now locate the least anisometric point on the femoral surface using any standard surgical tool equipped with optical bodies i.e. a drill.

Another method of a determination of anisometric points at the femur and the tibia is disclosed in the EP-A 0 603 089 CINQUIN. This known method comprises the use of reference bodies attached to the bones which comprise markers that can be detected by means of a optoelectronic position finder device. Furthermore, the known device comprises a pointer having markers as well so that the position of the pointer tip may be computed. For a selected point at the tibia a set of points at the femur is digitized by means of the pointer and then that point out of the set of points is computed which shows the most invariability in distance to the selected point at the tibia during flexion and extension of the knee joint.

The present invention intends to offer much more information needed to plan an anterior cruciate ligament reconstruction particularly it permits to display a surface section of the femur or of the tibia, the ligament and the planned drill holes. The extension of the ligament in case of knee flexion and extension may be digitized for any femoral or tibial attachment points and the drill holes may be planned such that they may be used as a drill guidance during the surgical operation.

The present invention solves the posed task by means of a device which comprises the features of claim 1.

A preferred embodiment of the device according to the present invention comprises reference bodies with at least three electromagnetic or acoustic waves emitting means attached, pointers having at least three electromagnetic or acoustic waves emitting means attached, a three-dimensional localizer device comprising at least two sensors or transmitters and a digitizing device used to determine the 3-dimensional coordinates of the electromagnetic or acoustic means by what means the position and the orientation of the reference bodies and the pointers or the pointer tips can be digitized by means of the three-dimensional localizer device and represented at the display of a computer and an image processing unit. The image processing unit generates a virtual three-dimensional surface at the display containing the points that were previously digitized by means of the pointers. The digitizing bases on the computation and analysis of electromagnetic wave interference patterns detected by at least three sensors that can be three linear charge-coupled device (CCD) cameras. Another possibility to compute the location of the reference bodies and the points bases on the videogrammetric analysis of the images received by at least two cameras that detect the electromagnetic waves emitted by said means. The image processing unit enables the generation of relevant elements such as a section of the femur or the tibia, the ligament or the femoral or tibial tunnels at or between determined points on the display. Furthermore, the image processing unit permits to display any desired and previously acquired relevant element or combination of these elements from any desired angle of observation, to display stationary or moving relevant elements and allows the representation of the ligament at the display during knee flexion and extension while the resulting extension is digitized.

Also possible is the representation of a dynamic view by using the image processing unit and an arthroscope.

In another embodiment the three-dimensional localizer device localizes the position and orientation of said pointers and said reference bodies by means of electromagnetic induction.

In yet another embodiment the at least three electromagnetic or acoustic waves emitting means are infrared light emitting diodes (IRED).

In a further embodiment the at least three electromagnetic or acoustic waves emitting means are optical reflectors.

In still a further embodiment the position and orientation of the pointer in relation to the reference bodies is determined by means of mechanic link devices.

A preferred method comprises the performance of the following steps:
a) attachment of reference bodies with electromagnetic or acoustic waves emitting means to the femur and to the tibia whereby the means attached to the reference bodies enable a computation of reference coordinate systems by digitizing that bases on the computation and analysis of electromagnetic wave interference patterns detected by at least three sensors that can be three linear charge-coupled device (CCD) cameras. Another possibility to compute the location of the reference bodies and the pointes bases on the videogrammetric analysis of the images received by at least two cameras that detect the electromagnetic waves emitted by said means;
b) a pointer is used to digitize points which are generated by placing the pointer tip at a desired position of the bones or of the bone joint and press a switch to localize the electromagnetic or acoustic waves emitting means attached at the pointer therewith digitizing the position and orientation of the pointer tip and said points are shown at the display of the computer related to a chosen reference system;
c) by means of an image processing unit a virtual three-dimensional surface at the display is generated containing the points that were previously determined by means of the pointers;
d) a three-dimensional representation of the ligament is generated by means of points that were previously determined by means of the pointers;
e) a three-dimensional simulation of the ligament during flexion-extension of the knee is shown at the display and the optimal locations of the ligament attachment points and of the drill holes are planned by the surgeon;
f) by means of the desirable angle of observation a medio-lateral view or a anterior-posterior view may be chosen; and
g) the different angle of observation enables the surgeon to plan the surgical operation also in view of a ligament impingement simulation.

Further advantageous embodiments of the invention are characterized in the dependent claims.

The invention and implementations of the invention will be disclosed more detailed in connection with the accompanying drawings in which:

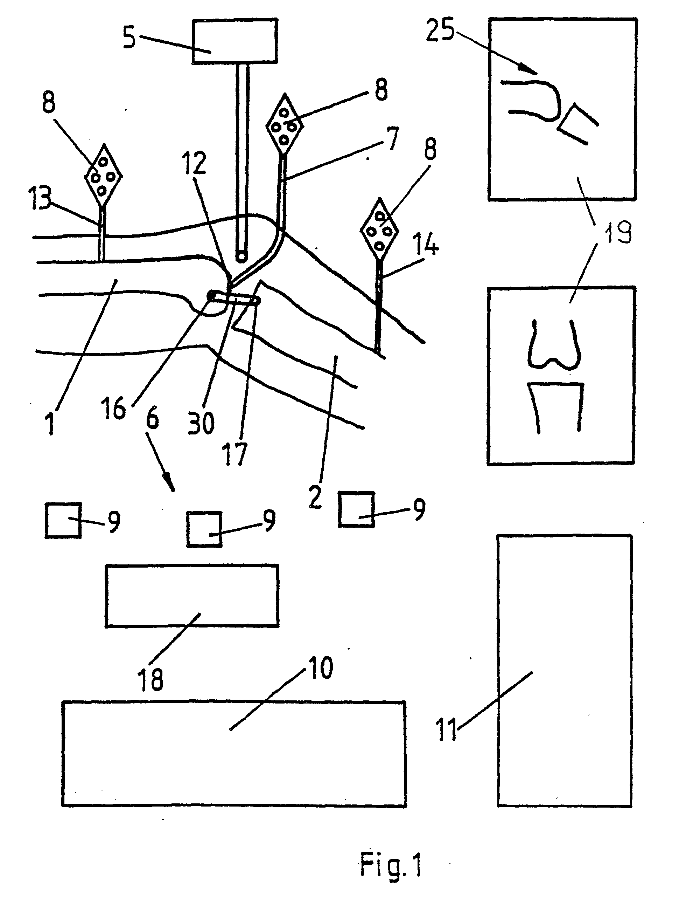

Fig. 1 shows the application of the device according to the invention in case of an anterior cruciate ligament reconstruction.

The device according to one implementation of the present invention is illustrated in Fig. 1. Device and method are applied for anterior cruciate ligament reconstruction. At the femur 1 and at the tibia 2 optical reference bodies 13;14 with Light Emitting Diodes as means 8 are attached each producing a reference system for use with the optical three-dimensional localizer device 6. This three-dimensional localizer device 6 comprises three linear charge-coupled device (CCD) cameras as sensors 9 and a digitizing device 18 used to determine the three-dimensional coordinates of the references 8. Since there are at least three LED's as means 8 attached at the reference bodies 13;14 and at the pointer 7 the position and the orientation of the reference bodies 13;14 and the pointers 7 or the pointer tip 12 can be determined by means of the three-dimensional localizer device (6) and three-dimensionally represented at the display 19 of the computer 11. An image processing unit (10) which is also comprised in the device according to the invention enables the generation of a virtual three-dimensional surface at the display 19 containing the points that were previously determined by means of the pointer 7. By using a pointer 7, an arthroscope 5 and the image processing unit 10 the surgeon determines relevant elements 25 (e.g. a section of the femur 1, a section of the tibia 2, the ligament, a drill hole etc.) at or between certain points on the display 19. By use of the image processing unit 10 any desired and previously acquired relevant element 25 or combination of these elements may be shown at any desired angle of observation at the display 19. For example a media-lateral view or a anterior-posterior view may be chosen. Furthermore, the image processing unit 10 allows the display of stationary or moving relevant elements 25 and enables a dynamic view by using an arthroscope 5. Moreover, the image processing unit enables the representation of the ligament at the display 19 during knee flexion and extension and the resulting extension of the ligament is digitized. The shifting to an anterior-posterior view also enables the surgeon to observe the ligament during knee flexion and extension and to perform a ligament impingement simulation. The tunnels 16;17 for the attachments of the ligament (30) as well as the ligament (30) are then determined at the display 19. By means of the drill holes at the display 19 the drill which must then also be provided with references 8 may be guided during the surgical operation.

## Claims

1. Device for planning a surgical operation by using virtual representation of a bone or a bone joint comprising
A) a computer (11) having a display (19);
B) reference bodies (13;14) with at least three electromagnetic or acoustic waves emitting means (8) attached;
C) moveable pointers (7) having at least three electromagnetic or acoustic waves emitting means (8) attached for digitizing points which are generated by placing the pointer tip (12) at a desired location of the bones or of the bone joint and localize the electromagnetic or acoustic waves emitting means (8) attached at the pointer (7) therewith digitizing the position and orientation of the pointer tip (12) and said points being shown at the display (19);
D) a three-dimensional localizer device (6) comprising at least two sensors or transmitters (9) and a digitizing device (18) used to determine the 3-dimensional coordinates of said electromagnetic or acoustic waves emitting means (8) by what means the position and the orientation of the reference bodies (13;14) and of the pointers (7) or the pointer tips (12) can be determined and represented on the display (19) of a computer (11);
E) an image processing unit (10); and
F) the image processing unit (10) generates a virtual three-dimensional surface on the display (19), said surface containing the points that were previously determined by means of the pointers (7);
**characterised in that**
G) the device further comprises an arthroscope (5) and that by means of this arthroscope (5) the image processing unit (10) generates an image at the display (19) representing the objects observed by means of the arthroscope (5) as relevant elements (25) as a section of the femur (1) or a section of the tibia (2); and
H) a virtual ligament and/or virtual drill holes at or between determined points on the display (19) are apt to be generated by means of the image processing unit (10) as relevant elements (25).

2. Device according to claim 1, **characterized in that** the image processing unit (10) enables to display any desired and previously acquired relevant element (25) or combination of these elements (25) from different viewing angles.

3. Device according to claim 1 or 2, **characterized in that** the image processing unit (10) enables to display stationary or moving relevant elements (25).

4. Device according to one of the claims 1 to 3, **characterized in that** the image processing unit (10) shows a three-dimensional representation of the connection between ligament attachment points (16;17) previously determined via the pointer (7) and represents therewith a ligament (30) on the display (19) during knee flexion and extension.

5. Device according to one of the claims 1 to 4, **characterised in that** said three-dimensional localizer device (6) localizes the position and orientation of said pointers (7) and said reference bodies (13;14) by means of electromagnetic induction.

6. Device according to one of the claims 1 to 5, **characterised in that** said at least three electromagnetic or acoustic waves emitting means (8) are optical light sources.

7. Device according to one of the claims 1 to 5, **characterised in that** said at least three electromagnetic or acoustic waves emitting means (8) are light emitting diodes (LED).

8. Device according to one of the claims 1 to 5, **characterised in that** said at least three electromagnetic or acoustic waves emitting means (8) are infrared light emitting diodes (IRED).

9. Device according to one of the claims 1 to 5, **characterised in that** said at least three electromagnetic or acoustic waves emitting means (8) are optical reflectors.

10. Device according to one of the claims 1 to 5, **characterised in that** said at least three electromagnetic or acoustic waves emitting means (8) are acoustical transmitters.

11. Device according to one of the claims 1 to 10, **characterized in that** said relevant elements (25) comprise the ligament, drill holes and previously digitized surfaces of the femur respectively the tibia.

12. Device according to claim 1, **characterized in that** the position and orientation of the pointer (7) in relation to the reference bodies (13;14) is determined by means of mechanic link devices.

## Patentansprüche

1. System zur Planung eines chirurgischen Eingriffs mit Unterstützung durch die virtuelle Darstellung eines Knochens oder Gelenks, bestehend aus
a) Einem Computer 11 mit Display 19.
b) Referenzkörpern 13 + 14, an denen mindestens drei Vorrichtungen zum Aussenden von elektromagnetischen oder Schallwellen 8 befestigt sind.
c) Beweglichen Pointern 7, an denen mindestens drei Vorrichtungen zum Aussenden von elektromagnetischen oder Schallwellen 8 zum Digitalisieren von Messpunkten, befestigt sind, wobei die Messpunkte erzeugt werden, indem die Pointerspitze 12 auf die entsprechenden Stellen von Knochen oder Gelenk platziert wird, wobei das elektromagnetischen oder Schallwellen erzeugende System 8, das am Pointer 7 befestigt ist, lokalisiert wird, sodass Position und Orientierung der Pointerspitze 12 digitalisiert werden und im Display 19 angezeigt werden.
d) Einem dreidimensional arbeiten Lokalisierungssystem 6 mit mindestens zwei Sensoren oder Transmittern 9 und einem Digitalisierungssystem 18 zur Bestimmung der dreidimensionalen Koordinaten des elektromagnetische oder Schallwellen erzeugenden Systems 8 bestimmt wird, sodass es möglich ist, Position und Orientierung der Referenzkörper 13 + 14 und der Pointer 7 oder Pointerspitzen 12 zu bestimmen und im Display 19 eines Computers 11 anzuzeigen.
e) Ein Bildverarbeitungssystem 10 und
f) Das Bildverarbeitungssystem 10 berechnet eine virtuelle dreidimensionale Oberfläche, die im Display 19 angezeigt wird, wobei die Oberfläche aus den Punkten besteht, die zuvor mit Hilfe der Pointer 7 erfasst wurde,
**dadurch gekennzeichnet, dass**
g) das System ebenfalls ein Arthroskop 5 enthält, und dass mittels des Arthroskops 5 das Bildverarbeitungssystem 10 ein Bild erzeugt, das im Display 19 angezeigt wird, das die Objekte , die mit Arthroskop 5 als relevante Elemente 25, als Schnitt durch Femur 1 oder Tibia 2 anzeigt und
h) ein virtuelles Ligament und/oder Bohrlöcher, an oder zwischen festgelegten Punkten im Display 19 durch das Bildverarbeitungssystem 10 als relevante Elemente 25 erzeugt werden können.

2. Einem System nach Anspruch 1, **dadurch gekennzeichnet, dass** das Bildverarbeitungssystem 10 die Darstellung beliebiger relevanter Elemente 25 oder Kombinationen dieser Elemente 25 aus verschiedenen Ansichtswinkeln ermöglicht, soweit die Elemente 25 zuvor erfasst wurden.

3. Einem System nach Ansprüchen 1 und 2, **dadurch gekennzeichnet, dass** das Bildverarbeitungssystem 10 die Anzeige stationärer oder beweglicher relevanter Elemente ermöglicht.

4. Einem System nach Ansprüchen 1 bis 3, **dadurch gekennzeichnet, dass** das Bildverarbeitungssystem 10 eine dreidimensionale Darstellung der Verbindung zwischen den Ligamentansatzpunkten 16 + 17, die zuvor mit dem Pointer 7 bestimmt wurden, anzeigt, und damit ein Ligament 30 im Display 19 bei Dehnung und Beugung des Knies anzeigt.

5. Einem System nach Ansprüchen 1 bis 4, **dadurch gekennzeichnet, dass** das beschriebene drei Vorrichtungen Lokalisierungssystem 6 Position und Orientierung der beschriebenen Pointer 7 und der beschriebenen Referenzkörper 13 + 14 mit elektromagnetischer Induktion lokalisiert.

6. Einem System nach Ansprüchen 1 bis 5, **dadurch gekennzeichnet, dass** es sich bei mindestens drei der elektromagnetischen oder Schallwellen erzeugenden Systeme 8 um optische Lichtquellen handelt.

7. Einem System nach Ansprüchen 1 bis 5, **dadurch gekennzeichnet, dass** es sich bei mindestens drei der elektromagnetischen oder Schallwellen erzeugenden Systeme 8 um Leuchtdioden (LEDs) handelt.

8. Einem System nach Ansprüchen 1 bis 5, **dadurch gekennzeichnet, dass** es sich bei mindestens drei der elektromagnetischen oder Schallwellen erzeugenden Systeme 8 um Infrarot-Leuchtdioden (IREDs) handelt.

9. Einem System nach Ansprüchen 1 bis 5, **dadurch gekennzeichnet, dass** es sich bei mindestens drei der elektromagnetischen oder Schallwellen erzeugenden Systeme 8 um optische Reflektoren handelt.

10. Einem System nach Ansprüchen 1 bis 5, **dadurch gekennzeichnet, dass** es sich bei mindestens drei der elektromagnetischen oder Schallwellen erzeugenden Systeme 8 um akustische Transmitter handelt.

11. Einem System nach Ansprüchen 1 bis 10, **dadurch gekennzeichnet, dass** es sich bei den beschriebenen relevanten Elementen um Ligament, Bohrlöcher und zuvor digitalisierte Oberflächen von Femur und Tibia handelt.

12. Einem System nach Anspruch 1, **dadurch gekennzeichnet, dass** Position und Orientierung des Pointers 7 in bezug auf die Referenzkörper mit Hilfe mechanischer Verbindungen bestimmt wird.

## Revendications

1. Dispositif pour planifier une opération chirurgicale en utilisant une représentation virtuelle d'un os ou d'une articulation, comprenant
A) un ordinateur (11) ayant un affichage (19),
B) des corps de référence (13, 14) avec au moins trois moyens émettant des ondes électromagnétiques ou acoustiques (8) qui y sont attachés,
C) des pointeurs (7) mobiles ayant au moins trois moyens émettant des ondes électromagnétiques ou acoustiques (8) qui y sont attachés pour numériser des points qui sont générés en plaçant la pointe (12) du pointeur à un endroit désiré des os ou de l'articulation et localisent les moyens émettant des ondes électromagnétiques ou acoustiques (8) attachés au pointeur (7) numérisant ainsi la position et l'orientation de la pointe (12) du pointeur et lesdits points étant représentés sur l'affichage (19),
D) un dispositif de localisation en trois dimensions (6) comprenant au moins deux capteurs ou transmetteurs (9) et un dispositif numériseur (18) utilisé pour déterminer les coordonnées en trois dimensions desdits moyens émettant des ondes électromagnétiques ou acoustiques (8) à l'aide desquels la position et l'orientation des corps de référence (13, 14) et des pointeurs (7) et des pointes (12) des pointeurs peuvent être déterminées et représentées sur l'affichage (19) d'un ordinateur (11),
E) une unité de traitement des images (10), et
F) l'unité de traitement des images (10) génère une surface virtuelle en trois dimensions sur l'affichage (19), ladite surface contenant les points qui ont été précédemment déterminés à l'aide des pointeurs (7),
**caractérisé en ce que**
G) le dispositif comprend en outre un arthroscope (5) et **en ce qu'**au moyen de cet arthroscope (5), l'unité de traitement des images (10) génère une image sur l'affichage (19) représentant les objets observés au moyen de l'arthroscope (5) en tant qu'éléments pertinents (25) d'une section du fémur (1) ou d'une section du tibia (2), et
H) un ligament virtuel et / ou des trous virtuels à percer en des points ou entre des points déterminés sur l'affichage (19) sont aptes à être générés à l'aide de l'unité de traitement des images (10) comme éléments pertinents (25).

2. Dispositif selon la revendication 1, **caractérisé en ce que** l'unité de traitement des images (10) permet d'afficher n'importe quel élément pertinent (25) souhaité et précédemment saisi ou n'importe quelle combinaison de ces éléments (25) sous différents angles de vue.

3. Dispositif selon la revendication 1 ou 2, **caractérisé en ce que** l'unité de traitement des images (10) permet d'afficher des éléments pertinents (25) stationnaires ou en mouvement.

4. Dispositif selon une des revendications 1 à 3, **caractérisé en ce que** l'unité de traitement des images (10) montre une représentation en trois dimensions de la connexion entre les points d'attache (16, 17) du ligament précédemment déterminés à l'aide du pointeur (7) et représente ainsi un ligament (30) sur l'affichage (19) pendant la flexion et l'extension du genou.

5. Dispositif selon une des revendications 1 à 4, **caractérisé en ce que** le dispositif de localisation en trois dimensions (6) localise la position et l'orientation desdits pointeurs (7) et desdits corps de référence (13, 14) au moyen de l'induction électromagnétique.

6. Dispositif selon une des revendications 1 à 5, **caractérisé en ce que** lesdits au moins trois moyens (8) émettant des ondes électromagnétiques ou acoustiques sont des sources de lumière optiques.

7. Dispositif selon une des revendications 1 à 5, **caractérisé en ce que** lesdits au moins trois moyens (8) émettant des ondes électromagnétiques ou acoustiques sont des diodes électroluminescentes (LED).

8. Dispositif selon une des revendications 1 à 5, **caractérisé en ce que** lesdits au moins trois moyens (8) émettant des ondes électromagnétiques ou acoustiques sont des diodes électroluminescentes infrarouges (IRED).

9. Dispositif selon une des revendications 1 à 5, **caractérisé en ce que** lesdits au moins trois moyens (8) émettant des ondes électromagnétiques ou acoustiques sont des réflecteurs optiques.

10. Dispositif selon une des revendications 1 à 5, **caractérisé en ce que** lesdits au moins trois moyens (8) émettant des ondes électromagnétiques ou acoustiques sont des transmetteurs acoustiques.

11. Dispositif selon une des revendications 1 à 10, **caractérisé en ce que** lesdits éléments pertinents (25) comprennent le ligament, les trous à percer et les surfaces précédemment numérisées du fémur et du tibia.

12. Dispositif selon la revendication 1, **caractérisé en ce que** la position et l'orientation du pointeur (7) par rapport aux corps de référence (13, 14) sont déterminées à l'aide de dispositifs de liaison mécaniques.
